# EUROPEAN PATENT APPLICATION

(11) **EP 4 084 585 A1**
(43) Date of publication of application: **02.11.2022**
(21) Application number: 20908023.3
(22) Date of filing: 22.12.2020
(51) Int. Cl.: H05B 47/115, H05B 47/16

(54) **SPECTRUM CONTROL SYSTEM, SPECTRUM CONTROL PROGRAM, AND SPECTRUM CONTROL METHOD**

(30) Priority: 27.12.2019 JP 2019239384
(71) Applicant: Kyocera Corporation, Kyoto-shi Kyoto 612-8501 (JP)
(72) Inventor: MITSUI, Katsuhiro, Kyoto-shi, Kyoto 612-8501 (JP)
(74) Representative: Viering, Jentschura & Partner mbB Patent- und Rechtsanwälte
(86) International application number: PCT/JP2020/048042
(87) International publication number: WO 2021/132282

(57) **Abstract**

A spectrum control system includes a storage unit that stores first information and second information related to the first information, an illumination device capable of controlling, based on the first information, a spectrum of light, a terminal device capable of acquiring second information, and a control unit communicably connected to the storage unit, the illumination device, and the terminal device. The control unit acquires second information from the terminal device, acquires first information related to the second information from the storage unit, and causes the illumination device to control, based on the first information, the spectrum.

## Description

### Cross-reference to Related Application

This application claims priority based on Japanese Patent Application No. 2019-239384 (filed December 27, 2019), the entire disclosure of which is hereby incorporated by reference.

### Technical Field

The present disclosure relates to a spectrum control system, a spectrum control program, and a spectrum control method.

### Background Art

A lighting control device that controls the dimming levels of a lighting apparatus to achieve a predetermined brightness is known (for example, see PTL 1).

### Citation List

### Patent Literature

PTL 1: Japanese Unexamined Patent Application Publication No. 2017-91776

### Summary of Invention

A spectrum control system according to an embodiment of the present disclosure includes a storage unit, an illumination device, a terminal device, and a control unit. The storage unit stores first information and second information related to the first information. The illumination device is capable of controlling, based on the first information, a spectrum of light. The terminal device is capable of acquiring the second information. The control unit is communicably connected to the storage unit, the illumination device, and the terminal device. The control unit acquires second information from the terminal device, and acquires first information related to the second information from the storage unit. The control unit causes the illumination device to control, based on the first information, the spectrum.

A spectrum control program according to an embodiment of the present disclosure causes a terminal device communicably connected to an illumination device that controls, based on first information, a spectrum of illuminating light for illuminating an illuminated space to execute a step of acquiring second information related to a user present in the illuminated space. The spectrum control program causes the terminal device to execute a step of acquiring, based on the second information, the first information. The spectrum control program causes the terminal device to execute a step of outputting the first information to the illumination device.

A spectrum control method according to an embodiment of the present disclosure includes a step of acquiring second information, a step of acquiring first information related to the second information, and a step of controlling, based on the first information, a spectrum of light.

### Brief Description of Drawings

[Fig. 1] Fig. 1 is a block diagram illustrating a configuration example of an illumination system according to an embodiment.
[Fig. 2] Fig. 2 is a table illustrating an example of spectrum data.
[Fig. 3] Fig. 3 is a table illustrating an example of spectrum data that changes in sequence over time.
[Fig. 4] Fig. 4 is a table illustrating an example of spectrum data that changes depending on the time of day.
[Fig. 5] Fig. 5 is a flowchart illustrating an example of a procedure of a spectrum control method executed by an illumination system.
[Fig. 6] Fig. 6 is a flowchart illustrating an example of a procedure of a spectrum control method executed by a terminal device.

### Description of Embodiments

It is desirable to illuminate a space where a user exists with illuminating light suited to the user.

According to a spectrum control system, a spectrum control program, and a spectrum control method according to an embodiment of the present disclosure, a space may be illuminated by illuminating light suited to a user.

An illumination device that can control the spectrum of illuminating light by selecting a spectrum from among a plurality of spectra is conceivable. In the case where data about the plurality of spectra is stored in advance in the illumination device, the illuminating light is light having one of the stored spectra. In this case, if increasing the number of selectable spectra is attempted, it is necessary for the illumination device to store data about more types of spectra.

An illumination device that allows a user to freely set the spectrum of illuminating light is conceivable. Here, there may be a case where a spectrum that is suited to the user is not set.

The present disclosure describes a spectrum control system, a spectrum control program, and a spectrum control method with which a space where a user spends time may be illuminated by illuminating light having a spectrum suited to the user.

### (System configuration example)

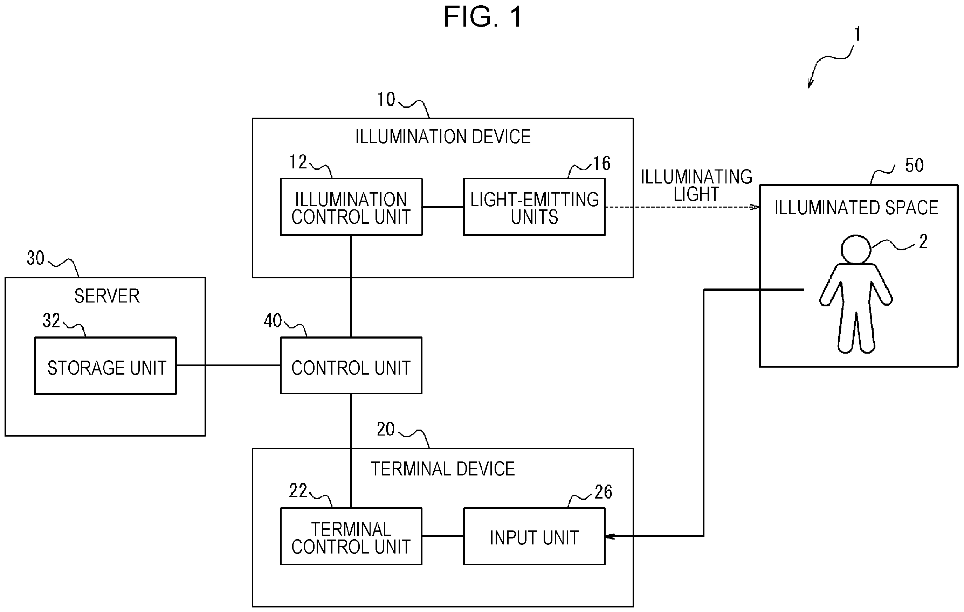

As illustrated in Fig. 1, a spectrum control system 1 according to an embodiment includes an illumination device 10, a terminal device 20, a storage unit 32, and a control unit 40. The illumination device 10 emits illuminating light that illuminates an illuminated space 50 where a user 2 is present. The illumination device 10 controls the spectrum that specifies the illuminating light. The control unit 40 outputs information for controlling the spectrum that specifies the illuminating light to be emitted by the illumination device 10. Information indicating the spectrum that specifies the illuminating light is also referred to as first information. The storage unit 32 stores the first information. The control unit 40 acquires the first information from the storage unit 32 and outputs the first information to the illumination device 10.

The storage unit 32 may include, for example, an electromagnetic storage medium such as a magnetic disk, or a memory such as semiconductor memory or magnetic memory. The storage unit 32 can store various information, programs to be executed by each constituent of the spectrum control system 1, and the like. The storage unit 32 may function as a working memory for each constituent of the spectrum control system 1.

The spectrum control system 1 may further include a server 30. In the case where the spectrum control system 1 further includes the server 30, the storage unit 32 is included in the server 30. The server 30 may include at least one processor. The processor included in the server 30 may be a processor that is the same as or similar to a processor included in the control unit 40. The storage unit 32 may be disposed in at least one configuration of the control unit 40, the server 30, the terminal device 20, or the illumination device 10. In other words, the storage unit 32 may be included in at least one of the control unit 40, the server 30, the terminal device 20, or the illumination device 10.

The control unit 40 may include at least one processor. The processor may execute a program that achieves various functions of an illumination control unit 12. The processor may be achieved as a single integrated circuit. An integrated circuit is also referred to as an IC. The processor may be achieved as a plurality of communicably connected integrated circuits and discrete circuits. The processor may be achieved based on various other known technologies.

The terminal device 20 includes a terminal control unit 22 and an input unit 26. The terminal control unit 22 outputs control instructions to each constituent of the terminal device 20 and acquires various information from each constituent. The terminal control unit 22 may include at least one processor to provide control and processing power for executing various functions. The processor included in the terminal control unit 22 may be a processor that is the same as or similar to a processor included in the control unit 40. The terminal control unit 22 may achieve at least a portion of the functions of the control unit 40. The terminal control unit 22 may include a terminal storage unit. The terminal storage unit of the terminal control unit 22 may be configured in a way that is the same as or similar to the storage unit 32. The terminal storage unit stores various information, programs to be executed by the terminal control unit 22, and the like. The terminal storage unit may function as a working memory of the terminal control unit 22. At least a portion of the terminal storage unit may be configured as a separate entity from the terminal control unit 22.

The input unit 26 may include an input device that accepts input from the user 2. The input device may include a touch panel, a keyboard, or a mouse, for example. The input unit 26 may include sensor, camera, or the like that detects the state of the user 2 or the state of the illuminated space 50. In other words, the input unit 26 may also acquire the state of the user 2 or the state of the illuminated space 50 through the sensor or the like. The sensor or the like may include, for example, a heart rate sensor that detects the heart rate of the user 2 as biological information about the user 2. The sensor or the like may include a temperature sensor that detects the temperature of the illuminated space 50 as environmental information about the illuminated space 50, for example. The input unit 26 is not limited to these examples, and may include a sensor that detects any of various types of parameters.

The terminal device 20 may also be achieved as a portion of the functions of a communication terminal such as a smartphone or a feature phone, a mobile personal computer (PC) such as a tablet PC or a laptop PC, or the like.

The illumination device 10 includes an illumination control unit 12 and a plurality of light-emitting units 16. Each light-emitting unit 16 emits light specified by a predetermined spectrum. The predetermined spectrum may have one or multiple peak wavelengths in a wavelength region from 360 nm to 780 nm. Light having a peak wavelength in the wavelength region from 360 nm to 780 nm is also referred to as visible light. The wavelength region from 360 nm to 780 nm is also referred to as the visible light region. In other words, the light-emitting units 16 may emit light specified by a spectrum having one or multiple peak wavelengths in the visible light region. The spectrum specifying the light is measured using spectroscopy, for example, with a spectrophotometer or the like.

Light that is the combination of the light emitted by the light-emitting units 16 is also referred to as combined light. The combined light is regarded as illuminating light emitted by the illumination device 10 as a whole. Among the plurality of light-emitting units 16, the spectrum of the light emitted by at least one light-emitting unit 16 is different from the spectrum of the light emitted by the other light-emitting unit(s) 16. The illumination control unit 12 can control the spectrum of the illuminating light emitted by the illumination device 10 as a whole by controlling each of the plurality of light-emitting units 16 that emit light in different spectra.

The illumination control unit 12 may include at least one processor to provide control and processing power for executing various functions of the illumination device 10. The processor included in the illumination control unit 12 may be a processor that is the same as or similar to a processor included in the control unit 40. The illumination control unit 12 may achieve at least a portion of the functions of the control unit 40.

The illumination control unit 12 may include an illumination storage unit. The illumination storage unit may be configured in a way that is the same as or similar to the storage unit 32. The illumination storage unit stores various information, programs to be executed by the illumination control unit 12, and the like. The illumination storage unit may function as a working memory of the illumination control unit 12. At least a portion of the illumination storage unit may be configured as a separate entity from the illumination control unit 12.

The light-emitting units 16 may include a light emitter and a wavelength conversion member, for example. The light emitter may emit light specified by a spectrum having a peak wavelength in a wavelength region from 360 nm to 430 nm, for example. Light specified by a spectrum having a peak wavelength in the wavelength region from 360 nm to 430 nm is also referred to as violet light. The wavelength region from 360 nm to 430 nm is also referred to as the violet light region. Visible light is taken to include violet light. The visible light region is taken to include the violet light region. The wavelength conversion member converts light entering the wavelength conversion member from the light emitter into light specified by a spectrum having a peak wavelength in the visible light region, and emits the converted light. The wavelength conversion member may also be considered to be excited by the light emitted by the light emitter and emit light of another wavelength. The light emitted by the light emitter is also referred to as excitation light. The excitation light is not limited to violet light, and may also be blue light specified by a spectrum having a peak wavelength in a wavelength region from 430 nm to 500 nm, for example.

The wavelength conversion member may include a phosphor. The phosphor may convert the excitation light into light specified by a spectrum having a peak wavelength in a wavelength region from 400 nm to 500 nm, or in other words into light of a blue color, for example. The phosphor may convert the excitation light into light specified by a spectrum having a peak wavelength in a wavelength region from 450 nm to 550 nm, or in other words into light of a bluish-green color, for example. The phosphor may convert the excitation light into light specified by a spectrum having a peak wavelength in a wavelength region from 500 nm to 600 nm, or in other words into light of a green color, for example. The phosphor may convert the excitation light into light specified by a spectrum having a peak wavelength in a wavelength region from 600 nm to 700 nm, or in other words into light of a red color, for example. The phosphor may convert the excitation light into light specified by a spectrum having a peak wavelength in a wavelength region from 680 nm to 800 nm, or in other words into near-infrared light, for example. The phosphor may also convert the excitation light into light of a vermilion color, a yellow color, or a white color, for example.

The wavelength conversion member may include multiple types of phosphors. The types of phosphors are not limited to the types described above, and may include other types. The combination of the types of phosphors contained in the wavelength conversion member is not particularly limited. The ratio of the phosphors contained in the wavelength conversion member is not particularly limited. The wavelength conversion member converts the excitation light into light specified by a spectrum determined based on the types and ratio of the contained phosphors. In the present embodiment, the illumination device 10 is assumed to include light-emitting units 16 that emit light of each of the following colors: violet, blue, green, bluish-green, vermilion, yellow, red, and white. The illumination device 10 controls the spectrum of the combined light by controlling the intensity of the light emitted by each light-emitting unit 16.

### <Example of first information>

As described above, the illumination device 10 is configured to control, based on the first information, the spectrum that specifies the illuminating light. The first information may include information that specifies the intensity of light to be emitted by each light-emitting unit 16. The light emitted by each light-emitting unit 16 corresponds to each color.

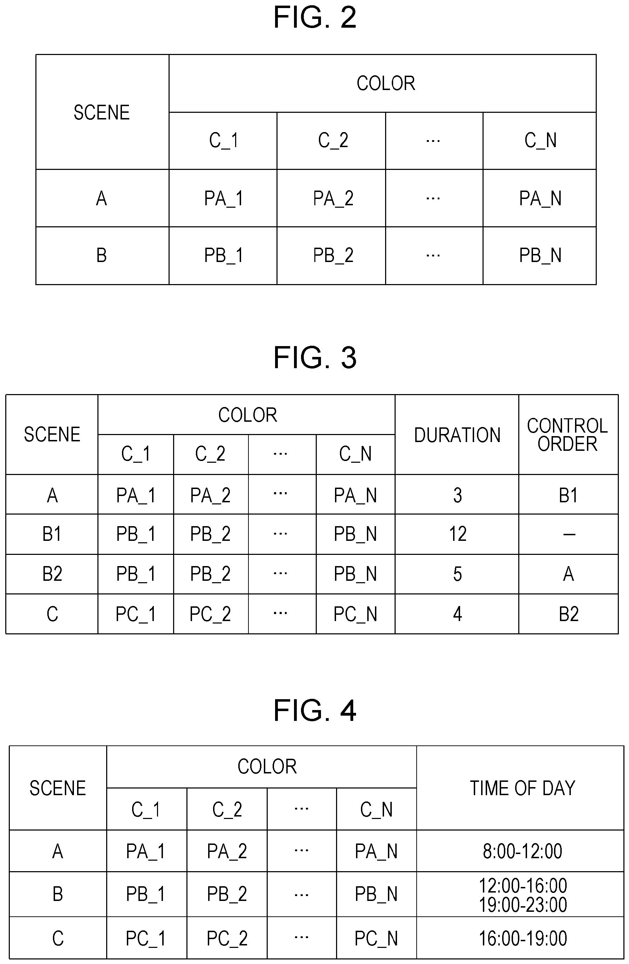

As illustrated in Fig. 2, the first information may be expressed as information that specifies the intensity of light corresponding to each color. The intensity of the light corresponding to each color is also referred to as the power of each color. Each row of the table corresponds to a type of illumination scene. The second row corresponds to an illumination scene indicated by A. The third row corresponds to an illumination scene indicated by B. Each column of the table corresponds to a type of color. The types of colors are indicated by the signs C_1 to C_N, where N is the number of colors that the illumination device 10 can emit. For example, the second column corresponds to the color indicated by C_1. N is taken to be a natural number equal to or greater than 2. If there are N colors (wavelength ranges) that the illumination device 10 can emit, there are N or more light-emitting units 16. Each cell of the table indicates the power of each color used to form the illumination scene. For example, among the colors used to form the illumination scene A, the power of the color indicated by C_1 is indicated by PA_1. Among the colors used to form the illumination scene B, the power of the color indicated by C_N is indicated by PB_N.

The control unit 40 outputs, to the illumination device 10, first information (for example, the table information illustrated in Fig. 1 or the like) indicating the power of each color. The illumination control unit 12 of the illumination device 10 controls the light-emitting units 16 based on the first information. As a result, the illumination device 10 can emit illuminating light specified by a spectrum according to the illumination scene designated by the control unit 40. It is also possible to control the power of each color to 0.

As illustrated in tables in Figs. 3 and 4, the first information may also include information associated with time.

In the table of Fig. 3, the sixth column indicates the duration of the control based on each illumination scene. The seventh column indicates the order in which the control based on each illumination scene is executed. The order in which the control is executed is also referred to as the control order. The illumination control unit 12 initially starts the control based on the illumination scene B1 for which the control order cell is blank (-), and continues the control for 12 hours. Following the control based on the illumination scene B1, the illumination control unit 12 starts the control based on the illumination scene A for which the control order cell contains B1, and continues the control for three hours. Following the control based on the illumination scene A, the illumination control unit 12 starts the control based on the illumination scene B2 for which the control order cell contains A, and continues the control for five hours. Following the control based on the illumination scene B2, the illumination control unit 12 starts the control based on the illumination scene C for which the control order cell contains B2, and continues the control for four hours. With this arrangement, the illumination device 10 can change the illuminating light over time. After the end of the control based on the illumination scene C, the illumination control unit 12 may start the control based on the illumination scene B1 again. With this arrangement, the illumination device 10 can change the illuminating light cyclically. The information indicating the order of control included in the first information is also referred to as a control pattern.

In the table of Fig. 3, the power of each color in the illumination scene B1 and the power of each color in the illumination scene B2 are set to the same value. With this arrangement, a configuration may be expressed in which a period for executing control based on the same illumination scene is included twice in a single control pattern. The number of types of illumination scenes included in the first information may be increased to match to the number of times the illumination scene is changed in the control pattern.

The expression of the order of the illumination scenes is not limited to the expression illustrated by way of example in Fig. 3. The order of the illumination scenes may also be indicated by a ranking, for example.

In the table of Fig. 4, the sixth column indicates the time of day of the control based on each illumination scene. In the period from 8:00 to 12:00, the illumination control unit 12 executes the control based on the illumination scene A. In the period from 12:00 to 16:00, the illumination control unit 12 executes the control based on the illumination scene B. In the period from 16:00 to 19:00, the illumination control unit 12 executes the control based on the illumination scene C. In the period from 19:00 to 23:00, the illumination control unit 12 executes the control based on the illumination scene B. With this arrangement, the illumination device 10 can change the illuminating light over time. In the table of Fig. 4, two times of day are associated as the times of day for executing the control based on the illumination scene B. With this arrangement, the rows indicating the control based on the illumination scene B are decreased to one row compared to Fig. 3. As a result, the data size of the first information may be reduced.

The first information may also include an illumination scene in which the power of all colors is 0, and thereby indicate a state in which illuminating light is not to be emitted.

In the example of Fig. 4, in the period from 23:00 to 8:00, the illumination control unit 12 controls the light-emitting units 16 such that illuminating light is not emitted. In other words, by including a time of day in which an illumination scene is not set, the first information may indicate that illuminating light is not to be emitted at that time of day. With this arrangement, the first information may indicate that illuminating light is not to be emitted without including an illumination scene in which the power of all colors is 0. As a result, the data size of the first information may be reduced.

### (Spectrum control example)

The terminal device 20 is configured to acquire information of at least one of information related to the user 2 or information related to the illuminated space 50. The information of at least one of information related to the user 2 or information related to the illuminated space 50 is also referred to as second information. The terminal device 20 outputs the second information to the control unit 40. The control unit 40 acquires the second information from the terminal device 20. The control unit 40 acquires the first information associated with the second information from the storage unit 32. The storage unit 32 is assumed to store the first information and the second information in association with each other. The control unit 40 outputs the first information acquired from the storage unit 32 to the illumination device 10. The illumination device 10 controls the light-emitting units 16 based on the first information, and controls the power of each color. By controlling the power of each color, the illumination device 10 can control the spectrum that specifies the illuminating light to be a spectrum based on information related to the user 2 or information related to the illuminated space 50, or in other words, the second information. As a result, the illumination device 10 can emit illuminating light suited to the user 2 present in the illuminated space 50.

### <Content of second information>

The second information may include information inputted by the user 2. The terminal device 20 may present a survey to the user 2 and accept a response inputted by the user 2 through the input unit 26. The survey may include, for example, questions related to the lifestyle of the user 2, such as when the user 2 sleeps, the content or timing of meals, and whether the user 2 drinks alcohol. The survey may also include questions related to symptoms the user 2 is aware of, such as areas of the body where the user 2 feels pain or how the user 2 feels. More specifically, a plurality of items are displayed on the terminal device 20, such as a mobile phone, for example, and by selecting any of the items, the spectrum is controlled based on optimal first information with respect to the selected item (second information).

The second information may also include biological information about the user 2. For example, the biological information about the user 2 may include, in relation to the user 2, heart rate, blood pressure, a sweating state or blood flow volume, an autonomic index, an activity level, and a period of no physical activity, as well as hormonal indicators such as stress. The biological information is not limited to the above and may also include various other information.

The second information may include environmental information about the illuminated space 50 where the user 2 is present. The environmental information about the illuminated space 50 may include, for example, the temperature or humidity, the amount of sunlight incident from the outside, or the carbon dioxide concentration of the illuminated space 50. The environmental information may also include the daylight hours, for example. Consequently, for example, in the winter, the illuminance or blue light power may be raised in the morning. In the summer, the illuminance or blue light power may be lowered in the evening. In this way, the illuminance or blue light power can also be adjusted to match the daylight hours. The environmental information is not limited to the above and may also include various other information.

The second information may also include information for estimating the physical state of the user 2. The control unit 40 may estimate the physical state of the user 2, based on information inputted by the user 2, biological information about the user 2, or environmental information about the illuminated space 50. The physical state of the user 2 may include a state indicating that the user 2 is afflicted with a predetermined disease or shows signs of a predetermined disease. The predetermined disease may include any of various types of diseases, such as a sleep disorder, dementia, depression, or diabetes, for example. The physical state of the user 2 may also include an obesity index of the user 2. The physical state of the user 2 may include states such as one indicating that the user 2 feels tense, the user 2 is concentrating, or the user 2 feels stressed. The physical state of the user 2 may include states such as one indicating that the user 2 feels sleepy, the user 2 is inattentive, or the user 2 is relaxed.

The second information may include information indicating present or future behavioral information about the user 2. The control unit 40 may specify behavioral information about the user 2, based on information inputted by the user 2. The control unit 40 may also estimate behavioral information about the user 2, based on information inputted by the user 2, biological information about the user 2, or environmental information about the illuminated space 50. The information indicating behavioral information about the user 2 may also include information indicating whether the user 2 is performing a simple task or information indicating that the user 2 is performing a creative task. The information indicating behavioral information about the user 2 may also include information indicating whether the user 2 is performing a creative task or information indicating that the user 2 is performing a task that requires concentration.

The second information may include information indicating emotional information about the user 2. The control unit 40 may specify emotional information about the user 2 based on information inputted by the user 2. Information indicating emotional information about the user 2 may include how good of a mood the user 2 is in, what colors the user 2 likes (such as warm colors or cool colors), and the like.

### <Generation of first information>

The control unit 40 acquires, based on the second information, the first information. The control unit 40 may acquire the first information associated with the second information from the storage unit 32. The control unit 40 may also transmit the second information to the server 30 and acquire first information generated based on the second information in the server 30. In this case, the server 30 receives the second information from the control unit 40, generates the first information based on the second information, and transmits the first information to the control unit 40.

The storage unit 32 stores in advance a table associating the first information and the second information. In the case where the server 30 includes the storage unit 32, the server 30 may generate the first information, based on the second information and the table.

### <<Example of table>>

The table stored in advance in the storage unit 32 may be prepared by an administrator of the spectrum control system 1. In the case where the storage unit 32 is included in the server 30, the server 30 accepts a registration of the first information by the administrator. The administrator associates spectra with states of the user 2 such that the illuminating light will induce an effect according to the state of the user 2. The administrator may be considered to be the subject who provides the first information.

For example, the administrator may register the first information by associating the second information corresponding to the case of the user 2 being afflicted with a predetermined disease with the first information indicating a spectrum that specifies illuminating light effective for treating the predetermined disease. The administrator may also register the first information only if there exists evidence proving a causal relationship between the illumination and an effect of treating the predetermined disease. The evidence may be issued by a qualified person such as a physician, for example. In the case where the administrator registers the first information on the condition that evidence exists, the first information may be considered to be authenticated in advance by the providing subject.

For example, the administrator may register the first information by associating the second information corresponding to the case of the user 2 being in a predetermined state with the first information indicating a spectrum that specifies illuminating light effective for getting out of the predetermined state. The predetermined state may include a state in which the user 2 feels sleepy or the concentration of the user 2 is lowered, for example. The administrator may also register the first information only if there exists evidence proving a causal relationship between the illumination and an effect of getting out of the predetermined state.

For example, the administrator may register the first information by associating the second information corresponding to the case where the user 2 needs to be in a predetermined state with the first information indicating a spectrum that specifies illuminating light effective for getting into the predetermined state. The predetermined state may include a state in which the user 2 can concentrate, for example. The predetermined state may also include a state in which the user 2 maintains a high level of concentration while remaining relaxed, also referred to as a flow state. The administrator may also register the first information only if there exists evidence proving a causal relationship between the illumination and an effect of getting into the predetermined state.

As described above, the spectrum control system 1 and terminal device 20 according to the present embodiment can illuminate the illuminated space 50 where the user 2 is present with illuminating light suited to the user 2.

### (Flowchart)

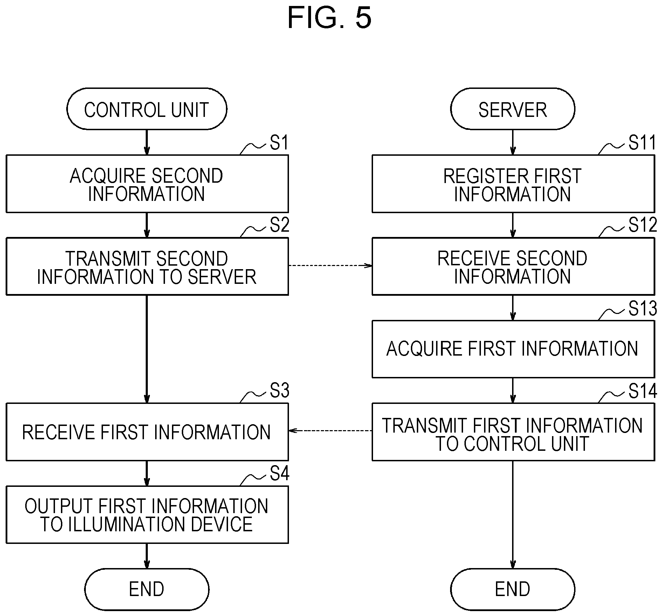

The spectrum control system 1 may execute a spectrum control method including the procedure of the flowchart illustrated by way of example in Fig. 5. The spectrum control method may also be achieved as a spectrum control program to be executed by a processor. In this example, the storage unit 32 is assumed to be included in the server 30.

The control unit 40 acquires the second information (step S1). The control unit 40 may acquire the second information from the terminal device 20. The terminal device 20 may accept the input of the second information from the user 2 through the input unit 26. If the input unit 26 includes a sensor, the terminal device 20 may acquire, as the second information, information related to the user 2 detected by the sensor. The terminal device 20 outputs the acquired second information to the control unit 40.

The server 30 accepts the registration of the first information (step S11). The server 30 registers the first information by causing the storage unit 32 to store the first information and the second information in association with each other. For example, the server 30 may cause the storage unit 32 to store the first information indicating a spectrum suited to the physical state of the user 2 in association with the second information corresponding to the physical state of the user 2. For example, the server 30 may cause the storage unit 32 to store the first information indicating a spectrum suited to the environment of the illuminated space 50 in association with the second information corresponding to the environment of the illuminated space 50.

The control unit 40 transmits the second information to the server 30 (step S2). The server 30 receives the second information (step S12).

The server 30 acquires, based on the second information, the first information (step S13). The server 30 may extract the first information associated with the acquired second information from among the first information that is registered by being stored in advance in the storage unit 32.

The server 30 transmits the first information to the control unit 40 (step S14). After the procedure in step S14, the server 30 ends the execution of the flowchart of Fig. 5. The control unit 40 receives the first information (step S3).

The control unit 40 outputs the first information to the illumination device 10 (step S15). After the procedure in step S4, the control unit 40 ends the execution of the flowchart of Fig. 5.

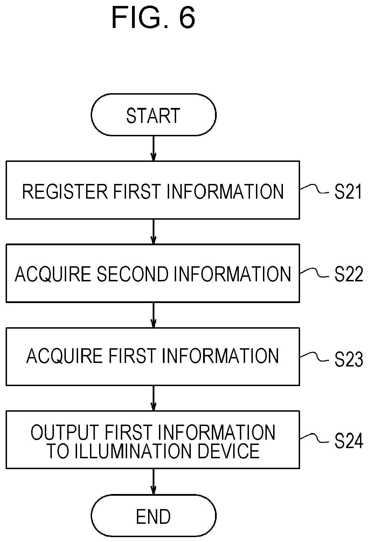

The control unit 40 may execute a spectrum control method including the procedure of the flowchart illustrated by way of example in Fig. 6. The spectrum control method may also be achieved as a spectrum control program to be executed by a processor. In this example, the storage unit 32 may be included in at least one of the control unit 40, the server 30, the terminal device 20, or the illumination device 10.

The control unit 40 accepts the registration of the first information (step S21). The control unit 40 may register the first information in the storage unit 32 by executing a procedure that is the same as or similar to the procedure in step S11 of Fig. 5 executed by the server 30.

The control unit 40 acquires the second information (step S22). The control unit 40 may executes a procedure that is the same as or similar to step S1 of Fig. 5.

The control unit 40 acquires, based on the second information, the first information (step S23). The control unit 40 may acquire the first information by executing a procedure that is the same as or similar to the procedure in step S13 of Fig. 5 executed by the server 30.

The control unit 40 outputs the first information to the illumination device 10 (step S24). After the procedure in step S24, the control unit 40 ends the execution of the flowchart of Fig. 6.

As described above, according to the spectrum control method according to the present embodiment, the illuminated space 50 where the user 2 is present may be illuminated by illuminating light suited to the user 2.

### <Illuminance>

The illumination device 10 may also be configured to control the illuminance as a parameter of the illuminating light. The illumination device 10 may also control the illuminance of visible light included in the illuminating light. The illumination device 10 may also control the spectrum to keep the illuminance constant. By controlling the spectrum with the illuminance as a parameter, the illumination device 10 can avoid discomforting the user 2 as much.

### <Color temperature>

The illumination device 10 may also be configured to control the color temperature as a parameter of the illuminating light. The color temperature is a parameter associated with the temperature of a black body. For a black body having a temperature expressed by T, the color temperature of the spectrum of light radiated by the black body is expressed by T. For example, the color temperature of the spectrum of light radiated by a black body at 5000 K (Kelvin) is expressed as 5000 K. The color of light having a color temperature of approximately 4000 K to 5000 K is also referred to as white. As the color temperature goes lower than white light, the color of the light may include more of a red component. In other words, light having a low color temperature appears reddish. As the color temperature goes higher than white light, the color of the light may include more of a blue component. In other words, light having a high color temperature appears bluish.

Not only the spectrum of light radiated by a black body but also a spectrum approximating the spectrum of light radiated by a black body may be expressed by the color temperature. In the case where a predetermined spectrum approximates the spectrum of light radiated by a black body having a color temperature expressed by T, the color temperature of the predetermined spectrum is assumed to be expressed by T. A determination of whether two spectra of light exist in an approximate relationship with each other may be made according to various conditions. The conditions under which two spectra of light exist in an approximate relationship with each other may include, for example, the condition that when the relative intensities of each wavelength are compared between the two spectra of light, the difference for each wavelength is within a predetermined range. The conditions under which two spectra of light exist in an approximate relationship with each other may also include, for example, the condition that the difference between peak wavelengths respectively included in the two spectra of light is within a predetermined range. The conditions under which two spectra of light exist in an approximate relationship are not limited to the above and may also include a variety of conditions.

For example, the spectrum of sunlight around noon may be approximated by the spectrum of light radiated by a black body at approximately 5000 K. In this case, the color temperature of sunlight around noon is assumed to be expressed as approximately 5000 K. The color of light expressed by a color temperature of approximately 5000 K is also referred to as neutral white. The color of light expressed by a color temperature of approximately 6500 K, higher than the color temperature of neutral white, is also referred to as daylight color. Daylight color includes more blue light components or components having wavelengths shorter than blue light compared to neutral white, and appears bluish. Conversely, neutral white appears to be a color closer to white than daylight color.

The illumination device 10 may also control the spectrum to raise the color temperature of the illuminating light. The illumination device 10 may also control the spectrum to lower the color temperature of the illuminating light. By controlling the spectrum based on the color temperature of the illuminating light, the illumination device 10 may induce the state of the user 2 to get into a predetermined state or get out of a predetermined state.

The illumination device 10 may also control the spectrum of the illuminating light to keep the color temperature constant as a parameter of the illuminating light. With this configuration, the user 2 is not discomforted as much.

### <Other embodiments>

As the spectrum control system 1 according to another embodiment according to the present invention, biological information about the user 2 and the first information of the illumination device 10 may be stored in a coordinated manner in the storage unit 32. In other words, a usage record of the first information of the illumination device 10 and the biological information when the first information (spectrum) is used may be stored in association with each other. Also, the usage record of the first information may not only include spectral information about the plurality of light-emitting units 16, but also the use times of specific spectra and the like. As a result, for example, in the case where the spectrum control system 1 additionally includes an AI, the usage record of the first information and the biological information can be used as training data to train the AI, and thereby control the illumination device 10 based on more optimal first information.

Also, as the spectrum control system 1 according to another embodiment, environmental information about the illuminated space 50 and the first information of the illumination device 10 may be stored in a coordinated manner in the storage unit 32. In other words, a usage record of the first information of the illumination device 10 and the environmental information when the first information (spectrum) is used may be stored in association with each other. Also, the usage record of the first information may not only include spectral information about the plurality of light-emitting units 16, but also the use times of specific spectra and the like. As a result, for example, in the case where the spectrum control system 1 additionally includes an AI, the usage record of the first information and the environmental information can be used as training data to train the AI, and thereby control the illumination device 10 based on more optimal first information. The spectrum control system 1 may also train the AI with the usage record of the first information, the biological information, and the environmental information.

The drawings used to describe an embodiment according to the present disclosure are schematic. The dimensional proportions and the like of the drawings do not necessarily match the real proportions.

An embodiment according to the present disclosure has been described based on the drawings and examples, but note that it would be easy for a person skilled in the art to make various modifications or revisions based on the present disclosure. Consequently, it should be understood that these modifications or revisions are included in the scope of the present disclosure. For example, the functions and the like included in each constituent and the like may be rearranged in logically non-contradictory ways, and it is possible to combine a plurality of constituents or the like into one or divide a constituent.

In the embodiment according to the present disclosure, the control unit 40, the illumination control unit 12, and the terminal control unit 22 are described as respectively separate configurations, but as an example, the control unit 40 may also be disposed in the illumination device 10 and additionally include the functions of the illumination control unit 12. Moreover, the control unit 40 may also be disposed in the terminal device 20 and additionally include the functions of the terminal control unit 22.

In the embodiment according to the present disclosure, an example in which a control order or durations are predetermined as fixed values is described as the first information, but the control order or the durations of the first information may also be variable values that change depending on the season, for example. In this case, for example, the daylight hours or the like may be acquired as the second information, and the duration of each spectrum may also be controlled by the control unit 40 such that the human circadian rhythm approaches a constant in conjunction with the length of the daylight hours.

Also, in the embodiment according to the present disclosure, for example, the storage unit 32, terminal device 20, or the like may be connected to an external server or the like and acquire external information such as a weather forecast.

In the present disclosure, qualifiers such as "first" and "second" are identifiers for distinguishing configurations. The numerals denoting the configurations distinguished by qualifiers such as "first" and "second" in the present disclosure can be exchanged. For example, the first information can exchange the identifiers "first" and "second" with the second information. The identifiers are exchanged at the same time. The configurations are still distinguished after the exchange of the identifiers. The identifiers may be removed. The configurations with the identifiers removed therefrom are distinguished by signs. The description of identifiers such as "first" and "second" in the present disclosure shall not be used as a basis for interpreting the order of the configurations or the existence of identifiers with smaller numbers.

### Reference Signs List

- 1: spectrum control system
- 2: user
- 10: illumination device (12: illumination control unit, 16: light-emitting unit)
- 20: terminal device (22: terminal control unit, 26: input unit)
- 30: server
- 32: storage unit
- 40: control unit
- 50: illuminated space

## Claims

1. A spectrum control system comprising:
a storage unit that stores first information and second information related to the first information;
an illumination device capable of controlling, based on the first information, a spectrum of light;
a terminal device capable of acquiring second information; and
a control unit communicably connected to the storage unit, the illumination device, and the terminal device, wherein
the control unit acquires second information from the terminal device, acquires first information related to the second information from the storage unit, and causes the illumination device to control, based on the first information, the spectrum.

2. The spectrum control system according to claim 1, wherein the first information includes information authenticated in advance by an entity providing the first information.

3. The spectrum control system according to claim 1 or 2, wherein the first information includes information associating the spectrum of light with time.

4. The spectrum control system according to any one of claims 1 to 3, wherein the second information includes biological information about a user to be illuminated by light.

5. The spectrum control system according to any one of claims 1 to 4, wherein the second information includes environmental information about a space to be illuminated by light.

6. The spectrum control system according to any one of claims 1 to 5, wherein the second information includes behavioral information about a user.

7. The spectrum control system according to any one of claims 1 to 6, wherein the illumination device is capable of additionally controlling an illuminance of light.

8. The spectrum control system according to any one of claims 1 to 7, wherein the illumination device is capable of additionally controlling a color temperature of light.

9. The spectrum control system according to any one of claims 1 to 8, wherein the illumination device is capable of controlling the spectrum while keeping a color temperature of light constant.

10. The spectrum control system according to any one of claims 1 to 9, wherein the storage unit is disposed in the illumination device.

11. The spectrum control system according to any one of claims 1 to 10, wherein the storage unit is disposed in the terminal device.

12. The spectrum control system according to any one of claims 1 to 11, further comprising:
a server communicably connected to the illumination device and the terminal device, wherein
the storage unit is disposed in the server.

13. A spectrum control program that causes a terminal device communicably connected to an illumination device that controls, based on first information, a spectrum of illuminating light for illuminating an illuminated space to execute:
a step of acquiring second information related to a user present in the illuminated space;
a step of acquiring, based on the second information, the first information; and
a step of outputting the first information to the illumination device.

14. A spectrum control method comprising:
a step of acquiring second information;
a step of acquiring first information related to the second information; and
a step of controlling, based on the first information, a spectrum of light.
